# EUROPEAN PATENT APPLICATION

(11) **EP 4 717 195 A2**
(43) Date of publication of application: **01.04.2026**
(21) Application number: 26151486.3
(22) Date of filing: 16.12.2020
(51) Int. Cl.: A61B 17/122

(54) **SYSTEM AND DEVICE FOR TREATING TISSUE**

(30) Priority: 23.04.2020 US 202016856890
(62) Divisional of application: 20842093.5
(71) Applicant: Boston Scientific Scimed, Inc., Maple Grove, Minnesota 55311 (US)
(72) Inventor: SAENZ VILLALOBOS, Gonzalo Jose, 40101 Fatima Heredia (CR); CALVO CAMACHO, Daniel, Ciudad Colon (CR); BERENZON, Rafael, 30303 La Union (CR); RYAN, Shawn, Littleton, Massachusetts 01460 (US)
(74) Representative: Vossius & Partner Patentanwälte Rechtsanwälte mbB

(57) **Abstract**

A device for treating tissue comprises a capsule extending longitudinally from a proximal end to a distal end and includes a channel extending therethrough along a longitudinal axis, and first and second clip arms. The first clip arm includes a first tissue retention feature and the second clip arm includes a second tissue retention feature. The first tooth of the first clip arm is positioned adjacent to a first lateral edge of the first clip arm while the second tooth of the first clip arm is positioned more centrally and separated from the first tooth of the first clip arm by a first gap having a width substantially corresponding to a width of a second sharpened tip of the second tooth of the second clip arm.

## Description

### Field

The present disclosure relates to a device, a system and a method for treating tissue. In particular, the present disclosure relates to a compression clip, a system and a method for delivering the clip to a target site through an endoscope to cause hemostasis of blood vessels along a gastrointestinal tract.

### Background

Pathologies of the gastrointestinal (GI) system, the biliary tree, the vascular system, and other body lumens and hollow organs are often treated through endoscopic procedures, many of which require hemostasis to control bleeding. Hemostasis clips grasp tissue surrounding a wound and hold edges of the wound together temporarily to allow natural healing processes to permanently close the wound. Specialized endoscopic clipping devices are used to deliver the clips at desired locations within the body after which the clip delivery device is withdrawn, leaving the clip within the body. In addition to providing hemostasis, endoscopic clipping devices may also be used to provide, for example, endoscopic marking and closure of luminal perforations, e.g., from endoscopic procedures involving access to internal tissue by passing a device through a wall of a natural body lumen.

The opening width of the clips usually determine a maximum size of a defect that it can potentially close. To close a defect larger than a maximum clip opening width, several sophisticated closure techniques have been developed that often require the additional use of snares, "8 rings", or other devices. As an alternative to these advanced and technically challenging methods, a novel technique for defect closure uses a technique known colloquially "hold and drag" that makes it possible to close sizable defects using only conventional clips. This technique simplifies the closure of large defects and reduces the time required for the procedure. However, the biggest challenge in using this technique is the high risk of tissue slipping out if a clip is reopened. As a result, it is not uncommon that many attempts are required for the successful closure of defects when applying this technique.

### Summary

The present disclosure relates to a device for treating tissue a capsule extending longitudinally from a proximal end to a distal end and including a channel extending therethrough along a longitudinal axis; and first and second clip arms, each extending from a proximal end to a distal end, the proximal ends being received within the channel of the capsule to be moved between an open tissue receiving configuration, in which the distal ends of the clip arms are separated from one another, and a closed tissue clipping configuration, in which the distal ends of the clip arms are moved toward one another.

The first clip arm includes a first portion which, in the tissue clipping configuration extends substantially parallel to a first portion of the second arm and parallel to the longitudinal axis, the first clip arm including a first tissue retention feature extending radially inward from the first portion of the first clip arm toward the longitudinal axis, the first tissue retention feature being configured to grip target tissue when in the tissue clipping configuration and subsequently release the target tissue when the first and second clip arms are moved toward the open tissue receiving configuration. The first retention feature is coupled to the first portion of the first clip arm via a first curved portion and wherein the second clip arm includes a second tissue retention feature extending radially inward from the first portion of the second clip arm toward the longitudinal axis. The second tissue retention feature is configured to grip target tissue when in the tissue clipping configuration and subsequently release the target tissue when the first and second clip arms are moved toward the open tissue receiving configuration. The second retention feature is coupled to the first portion of the second clip arm via a second curved portion.

In an embodiment, the first tissue retention feature includes a first tooth extending radially inward from the first curved portion and the second tissue retention feature includes a second tooth extending radially inward toward the longitudinal axis from the second curved portion.

In an embodiment, the first retention feature has a number of teeth that differs from a number of teeth of the second retention feature.

In an embodiment, the first retention feature includes a first guard positioned and shaped to overhang a sharpened tip of the second tooth so that the sharpened tip of the second tooth is, when the first and second clip arms are in the tissue clipping configuration, proximal of the first guard and closer to the longitudinal axis than lateral edges of the first and second clip arms.

In an embodiment, the second retention feature includes a second guard positioned and shaped to overhang a sharpened tip of the first tooth so that the sharpened tip of the first tooth is, when the first and second clip arms are in the tissue clipping configuration, proximal of the second guard and closer to the longitudinal axis than lateral edges of the first and second clip arms.

In an embodiment, the first retention feature extends at a first angle relative to the first portion of the first clip arm of 30 degrees to 100 degrees and the second retention feature extends at a second angle relative to the first portion of the second clip arm of 30 degrees to 100 degrees.

In an embodiment, the first angle is equal to the second angle.

In an embodiment, the first retention feature includes first sharpened tips extending radially inward therefrom and the second retention feature includes second sharpened tips extending radially inward therefrom.

In an embodiment, the first sharpened tips and the second sharpened tips are one of triangular, circular, or rectangular in shape.

In an embodiment, the first retention feature includes a first plurality of barbs extending radially inward therefrom and the second retention feature includes a second plurality of barbs extending radially inward therefrom.

The present disclosure also relates to a reloadable clip system which includes an applicator including a catheter and a control member extending therethrough, the control member extending from a proximal end to a distal end and being longitudinally movable relative to the applicator; at least one clip assembly coupled to the applicator. Each clip assembly includes: a capsule extending longitudinally from a proximal end to a distal end and including a channel extending therethrough along a longitudinal axis; and first and second clip arms each extending from a proximal end to a distal end, the proximal ends being received within the channel of the capsule to be moved between an open tissue receiving configuration, in which the distal ends of the clip arms are separated from one another, and a closed tissue clipping configuration, in which the distal ends of the clip arms are moved toward one another.

The first clip arm includes a first portion which, in the tissue clipping configuration extends substantially parallel to a first portion of the second arm and parallel to the longitudinal axis, the first clip arm including a first tissue retention feature extending radially inward from the first portion of the first clip arm toward the longitudinal axis. The first tissue retention feature is configured to grip target tissue when in the tissue clipping configuration and subsequently release the target tissue when the first and second clip arms are moved toward the open tissue receiving configuration, the first retention feature being coupled to the first portion of the first clip arm via a first curved portion and wherein the second clip arm includes a second tissue retention feature extending radially inward from the first portion of the second clip arm toward the longitudinal axis. The second tissue retention feature is configured to grip target tissue when in the tissue clipping configuration and subsequently release the target tissue when the first and second clip arms are moved toward the open tissue receiving configuration. The second retention feature is coupled to the first portion of the second clip arm via a second curved portion.

In an embodiment, the first tissue retention feature includes a first tooth extending radially inward from the first curved portion and the second tissue retention feature includes a second tooth extending radially inward toward the longitudinal axis from the second curved portion.

In an embodiment, the first retention feature includes a first guard positioned and shaped to overhang a sharpened tip of the second tooth so that the sharpened tip of the second tooth is, when the first and second clip arms are in the tissue clipping configuration, proximal of the first guard and closer to the longitudinal axis than lateral edges of the first and second clip arms.

In an embodiment, the first retention feature extends at a first angle relative to the first portion of the first clip arm of 30 degrees to 100 degrees and the second retention feature extends at a second angle relative to the first portion of the second clip arm of 30 degrees to 100 degrees.

In an embodiment, the second retention feature includes a second guard positioned and shaped to overhang a sharpened tip of the first tooth so that the sharpened tip of the first tooth is, when the first and second clip arms are in the tissue clipping configuration, proximal of the second guard and closer to the longitudinal axis than lateral edges of the first and second clip arms.

In addition, the present disclosure relates to a method for treating tissue which includes inserting a first clip assembly to a target site within a living body via a working channel of an endoscope, the first clip assembly including a capsule extending longitudinally from a proximal end to a distal end and including a channel extending therethrough along a longitudinal axis and first and second clip arms slidably receive within the capsule to be movable between an open configuration, in which distal ends of the clip arms are separated from one another, and a closed configuration, in which distal ends of the clip arms are drawn toward on another; positioning the clip arms in contact with a first target tissue; moving the first clip assembly from the open configuration to the closed configuration such that a first tissue retention feature extending radially inward from the first portion of the first clip arm toward the longitudinal axis and a second tissue retention feature extending radially inward from a first portion of the second clip arm toward the longitudinal axis pierce through the first target tissue; moving the first clip assembly from the closed configuration to the open configuration, the first tissue retention feature releasing the first target tissue while the second tissue retention feature retains the first target tissue thereon; positioning the clip arms in contact with a second target tissue; and moving the first clip assembly from the open configuration to the closed configuration to clip the first and second target tissues between the first and second clip arms.

In an embodiment, the first tissue retention feature includes a first tooth extending radially inward from a first curved portion that couples the first retention feature to the first portion of the first clip arm and the second tissue retention feature includes a second tooth extending radially inward toward the longitudinal axis from a second curved portion that couples the second retention feature to the first portion of the second clip arm.

In an embodiment, the first retention feature includes a first guard positioned and shaped to overhang a sharpened tip of the second tooth so that the sharpened tip of the second tooth is, when the first and second clip arms are in the tissue clipping configuration, proximal of the first guard and closer to the longitudinal axis than lateral edges of the first and second clip arms.

In an embodiment, the method further includes loading a first clip assembly on an applicator by coupling a control member of the applicator to the proximal ends of clip arms.

In an embodiment, the method further includes releasing the clip assembly from the applicator; and loading a second clip assembly on an applicator by coupling a control member of the applicator to the proximal ends of clip arms.

### Brief Description

Fig. 1 shows a top plan view of a clip system according to an exemplary embodiment of the present disclosure.
Fig. 2 shows a front view of a clip assembly of the clip system of Fig. 1.
Fig. 3 shows a perspective view of distal ends of clip arms of the clip assembly of the clip system of Fig. 1.
Fig. 4 shows a perspective view of the clip assembly of the clip system of Fig. 1.
Fig. 5 shows a top plan view of a clip system according to a second exemplary embodiment of the present disclosure.
Fig. 6 shows a front view of a clip assembly of the clip system of Fig. 5.
Fig. 7 shows a top plan view of a clip system according to a third exemplary embodiment of the present disclosure.
Fig. 8 shows a front view of a clip assembly of the clip system of Fig. 7.
Fig. 9 shows a perspective view of distal ends of clip arms of the clip assembly of the clip system of Fig. 7.
Fig. 10 shows a top plan view of a clip system according to a fourth exemplary embodiment of the present disclosure.
Fig. 11 shows a front view of a clip assembly of the clip system of Fig. 10.
Fig. 12 shows a side view of the distal end of a clip arm of the clip assembly of the clip system of Fig. 10.
Fig. 13 shows a first step of a method of use of the clip system of Fig. 1.
Fig. 14 shows a second step of the method of use of the clip system of Fig. 1.
Fig. 15 shows a third step of the method of use of the clip system of Fig. 1.
Fig. 16 shows a fourth step of the method of use of the clip system of Fig. 1.
Fig. 17 shows a top plan view of a clip system according to a fifth exemplary embodiment of the present disclosure.
Fig. 18 shows a perspective view of a clip arm of a clip assembly of the clip system of Fig. 17.
Figs. 19A-19B show perspective views of a distal end of a clip arm of the clip assembly of the clip system of Fig. 17.
Fig. 20 shows a perspective view of the clip assembly of the clip system of Fig. 17.
Fig. 21A shows a perspective view of distal ends of the clip arms the clip assembly of the clip system of Fig. 17.
Fig. 21B shows a front view of a first clip arm of the clip assembly of the clip system of Fig. 17.
Fig. 21C shows a front view of a second clip arm of the clip assembly of the clip system of Fig. 17.
Fig. 22 shows a front view of a distal end of a clip arm of the clip assembly of the clip system of Fig. 17.
Fig. 23 shows a front view of a distal end of a clip arm of the clip assembly according to further embodiments of the clip system of Fig. 17.
Fig. 24 shows a front view of a distal end of a clip arm of the clip assembly according to a further embodiment of the clip system of Fig. 17.
Fig. 25 shows a top plan view of a clip system according to a sixth exemplary embodiment of the present disclosure.
Fig. 26A shows a perspective view of distal ends of the clip arms of the clip assembly of the clip system of Fig. 25.
Fig. 26B shows a front view of a first clip arm of the clip assembly of the clip system of Fig. 25.
Fig. 26C shows a front view of a second clip arm of the clip assembly of the clip system of Fig. 25.

### Detailed Description

The present disclosure may be further understood with reference to the following description and the appended drawings, wherein like elements are referred to with the same reference numerals. The present disclosure relates to a clipping system and, in particular, relates to a single use or reloadable endoscopic clipping system for treating tissue perforations, defects and/or bleeds. Exemplary embodiments of the present disclosure describe a hemostatic clip having aggressive gripping or retention features to improve attachment to target tissue when the clip is at least partially in an open configuration. In particular, exemplary embodiments describe a hemostatic clip with clip arms having spikes, hooks, barbs and other geometries as gripping features to allow grabbing and holding of tissue by a single clip arm. It should be noted that the terms "proximal" and "distal," as used herein, are intended to refer to toward (proximal) and away from (distal) a user of the device.

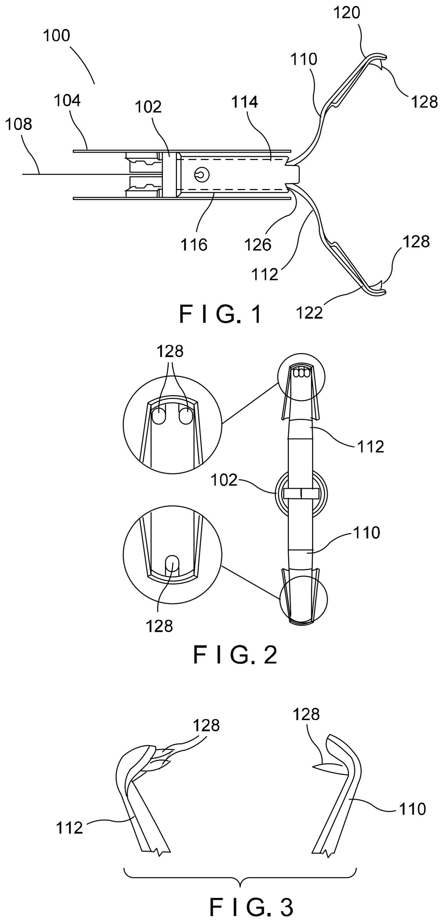

As shown in Figs. 1, a system 100 according to an exemplary embodiment of the present disclosure comprises a clip assembly 102 insertable into a living body through, for example, a working channel of an endoscope to target tissue to be treated. The clip assembly 102 is sufficiently flexible to permit it to traverse a tortuous path through the body - e.g., passing through the working channel of a flexible endoscope inserted through a natural body lumen accessed via a natural bodily orifice.

In an exemplary embodiment, the clip assembly 102 is loadable onto a distal portion of an applicator 104 prior to insertion of the clip assembly 102 into a living body for the clipping of target tissue. The applicator 104 and the clip assembly 102 are configured so that, after deployment of the clip assembly 102 in the living body, a new clip assembly 102 may be loaded onto the applicator 104 so that the same applicator 104 may be used to deliver a new clip assembly 102 to a second portion of target tissue in the living body. The applicator 104 may include a catheter (not shown) and a control member 108 extending therethrough. The clip assembly 102 includes first and second clip arms 110, 112 slidably received within a longitudinal channel 114 of a capsule 116.

The first and second clip arms 110, 112 can be moved between an open tissue receiving configuration, in which the distal ends 120, 122 of the clip arms 110, 112, respectively, are separated from one another to receive target tissue therebetween, and a closed tissue gripping configuration, in which the distal ends 120, 122 of the clip arms 110, 112 are moved toward one another to grip the target tissue therebetween. The clip arms 110, 112 are movable between the open and the closed configurations via the control member 108 extending into the capsule 116. A proximal end (not shown) of the control member 108 is connected to an actuator on a handle positioned outside of the human body. In this embodiment, a distal end of the control member 108 is coupled to proximal ends of the clip arms 110, 112.

As further shown in Fig. 1, the clip arms 110, 112 according to an exemplary embodiment of the present disclosure which extend from proximal ends (not shown) to the distal ends 120, 122. As those skilled in the art will understand, arms 110, 112 of this embodiment are biased toward an open tissue receiving position in which the distal ends 120, 122 are separated from one another to receive tissue. That is, the arms 110, 112 are formed so that they spring open to the tissue receiving position when they are moved distally out of the constraint of the capsule 116. However, as would be understood by those skilled in the art, the clip assembly 102 may include a separate member urging the clip arms 110, 112 away from one another in addition, or as an alternative, to this bias.

In an exemplary embodiment, the clip arms 110, 112 may include locking tabs (not shown) at their proximal ends for engaging distal components within the capsule 116 when the clip assembly 102 has been deployed. Locking tabs (not shown) may also form a mechanical lock with windows formed on lateral sides of the capsule 116 after the clip has been deployed. However, those skilled in the art will understand that any of the various known mechanisms for releasably coupling a clip assembly 102 (e.g., a capsule including clip arms) to an insertion device may be employed without deviating from the scope of the invention.

As indicated above, the clip arms 110, 112 are biased toward the open configuration so that, when the clip arms 110, 112 are moved distally past a distal end 126 of the capsule 116, distal ends 120, 122 of the clip arms 110, 112 separate from one another to the open configuration. When the clip arms 110, 112 are drawn proximally into the capsule 116, contact with the capsule 116 draws the clip arms 110, 112 toward one another to the closed configuration. Upon deployment, the clip arms 110, 112 are locked within the capsule 116 which holds them in the closed position as would be understood by those skilled in the art. As described above, the clip arms 110, 112 are moved between the open and closed configurations via the control member 108, which is moved proximally and distally relative to the capsule 116 via an actuator at a handle that remains outside the body.

In an exemplary embodiment, one or both of the clip arms 110, 112 may include spikes 128 extending from an interior side of a distal tip 130 of the clip arms 110, 112. As shown in Figs. 2-3, the first clip arm 110 has a single spike 128 while the second clip arm 112 has two spikes 128. The spikes 128 extend substantially perpendicularly from the interior face of the clip arms 110, 112 radially inward toward the other of the clip arms 110, 112.

In an exemplary embodiment, the spikes 128 are designed to pierce or at least engage the tissue such that the tension in the tissue (from pulling it across the defect) creates more engagement between the spikes 128 and the tissue. For example, a fishhook shape demonstrates this engagement once it pierces the tissue: application of additional force in the same direction pushes the tissue further up the hook while the direction of force must be reversed to remove the hook.

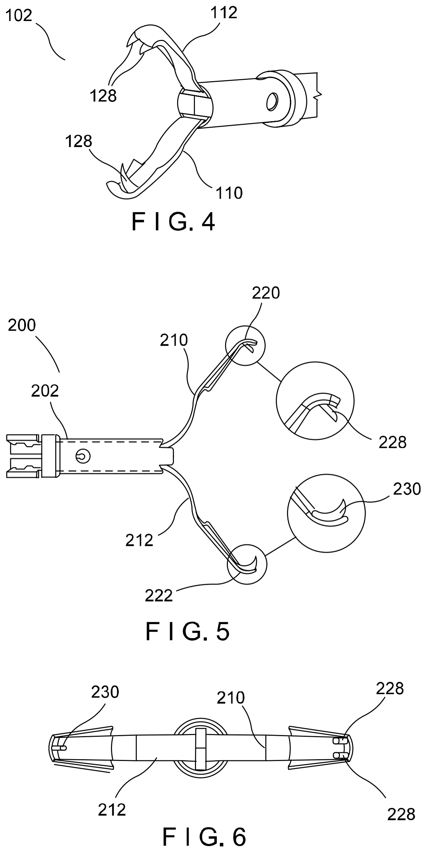

As can be seen in Fig. 3, the spikes 128 on opposing arms 110, 112 are offset from one another longitudinally so that they do not prevent complete closure of the clip arms 110, 112 around the tissue defect. For example, as shown in Fig. 3, the spike 128 of the first clip arm 110 is positioned one of distally or proximally of the two spikes 128 on the second clip arm 112 when the clip assembly 102 is in the closed configuration. Furthermore, as shown in Fig. 4, the spikes of the first and second clip arms 110, 112 are offset from one another laterally so that the spike 128 of the first clip arm 110 is received between the spikes 128 of the second clip arm 112 without any interference.

In an alternate embodiment, the spikes 128 are configured to extend in different planes to be received by an opposing arm without interference from the spike 128 of the other arm. For example, the spike 128 of the first clip arm 110 is positioned so that it fits between the two spikes 128 of the second clip arm 112 when the clip assembly 102 is in the closed configuration. In another embodiment, both of the spikes 128 are configured to extend in substantially the same plane so that when arms 110, 112 are in a closed configuration ends of the spikes 128 from opposing arms 110, 112 touch one another.

As would be understood by those skilled in the art, the spikes 128 are sufficiently sharp to pierce through tissue and hook therein. In an embodiment, the spikes 128 of the second clip arm 112 are angled to a greater degree with respect to the rest of the clip arm 112 so that the spikes 128 remain hooked within the target tissue when the clip assembly 102 is moved to the open configuration to capture another portion of tissue. Specifically, the spikes 128 may likewise extend from the interior face of clip arm 112 at various angles between 20 and 90 degrees relative to the longitudinal axis L of the clip arm 112 to allow the spikes 128 to continue to grab the tissue when the clip arms 110, 112 are in the open configuration.

In use, the operator will orient the clip arms 110, 112 so that the second clip arm 112 will be on the outside of the tissue defect and the first clip arm 110 is on the inside of the tissue defect. Next, the operator will close the clip assembly 102 on the edge of the tissue defect and the spikes 128 will pierce through the mucosal layer to keep the tissue inside the clip arms 110, 112. When the clip assembly 102 is dragged to the opposite edge of the tissue defect and gently reopened, the tissue flap will still be hooked onto the second clip arm 112 but released from the first arm 110. That is, the direction of force of the first clip arm 110 is reversed so that the tissue is unclipped from the first clip arm 110 while remaining on the second clip arm 112.

The first clip arm 110 can then be used to draw the opposite edge of the defect inside the clip assembly 102. The spikes 128 on both jaws 110, 112 will ensure that the edges of the defect are securely captured and will not slip out when the clip is closed and deployed. When the two opposing edges of the tissue defect are connected by the clip assembly 102, the clip assembly 102 will be released from the applicator 104 and further clips can be placed along the tissue defect to finalize the closure, as will be described in further detail below.

In another exemplary embodiment, shown in Figs. 5-6, a system 200 is substantially the same as system 100 except as described herein. The system 200 includes a clip assembly 202 with first and second clip arms 210, 212. In this embodiment, the clip arms 210, 212 use a combination of hooks and spikes to retain a first tissue edge on one arm while dragging a second tissue edge toward the first edge with the second arm. Specifically, as shown in Fig. 5, the first arm 210 includes two spikes 228, similar to the spikes 128 of the clip assembly 102, extending substantially perpendicularly from the interior face of the clip arm 210 radially inward toward the second clip arm 212. The second arm 212 has a hooked distal end 222 with a sharp distal-most tip 230 configured to pierce and retain tissue thereon.

In an exemplary embodiment, the distal tip 230 is angled at approximately 90 to 180 degrees relative to the rest of the clip arm 212. For example, in an embodiment, the distal end 222 of the second clip arm 212 may be bent back toward the clip assembly 202 such the distal tip 230 is approximately parallel to the rest of the clip arm 212. This embodiment provides a high degree of tissue retention once the distal tip 230 has pierced the tissue defect. In another example, the distal end 222 may have a greater than 90 degrees bend such that distal tip is substantially perpendicular to the proximal portion of the clip arm 212. This embodiment allows the tissue defect to be more easily pierced by the distal tip 230.

In use, the hooked distal end 222 of the second clip arm 212, as discussed above, is designed to adhere to the tissue and keep a first edge of the tissue defect inside the clip arms 210, 212 while the spike 228 of the first clip arm 210 is designed to release the first edge of the tissue defect and draw the opposite second edge of the tissue defect in when the clip assembly 202 is reopened. This combination of aggressive features has demonstrated to be especially effective for the "hold and drag" technique.

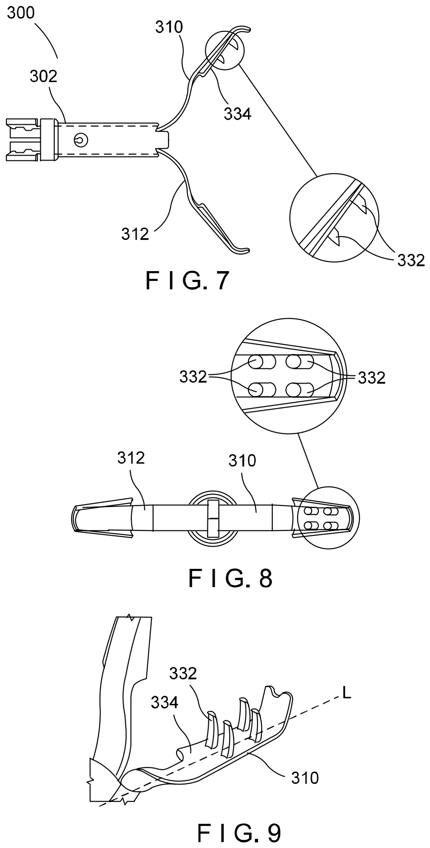

In another exemplary embodiment, shown in Figs. 7-9, a system 300 is substantially the same as systems 100, 200 except as described herein. The system 300 includes a clip assembly 302 with first and second clip arms 310, 312. The first clip arm 310, in this embodiment, includes one or more barbs 332 attached to an interior face 334 of a distal portion of the first clip arm 310. Clip arms with barbs are especially effective if the operator can force the barbed arm flat against the target tissue. This positioning of the arm flat against the tissue allows each of the barbs 332 to pierce the submucosal tissue layer and force the tissue to stay attached to the clip arm even under high tension.

As shown in Figs. 8-9, the first clip arm 310 of this embodiment includes four barbs 332 which extend substantially perpendicularly from the interior face 334 of the first clip arm 310 radially inward toward the second clip arm 312. It will be understood that while the present embodiment includes four barbs 332, any number of barbs may be used. The barbs 334 may likewise extend from the interior face 334 at various angles between 20 and 90 degrees and, more particularly, 45 degrees relative to the longitudinal axis L of the clip arm 310 to allow the barbs 332 to grab tissue when the clip arms 310, 312 are in the open configuration. That is, the barbs 332 may extend outward from the interior face 334 of the first clip arm 310 in a plane substantially perpendicular to a plane containing the interior face 334 of the first clip arm 310 or may extend at an angle and thus in a plane that is not perpendicular to the plane containing the interior face 334 of the clip arm 310.

In an embodiment, shown in Figs. 8-9 the barbs 332 are positioned on the interior face 334 as pairs that are substantially parallel with one another. However, it is understood that the barbs 332 may take any configuration such as, for example, longitudinally staggered pairs, a single longitudinal line along a central longitudinal axis of the clip arm 310, etc. As can be seen in the figures, the second arm 312 has a flat profile along its length.

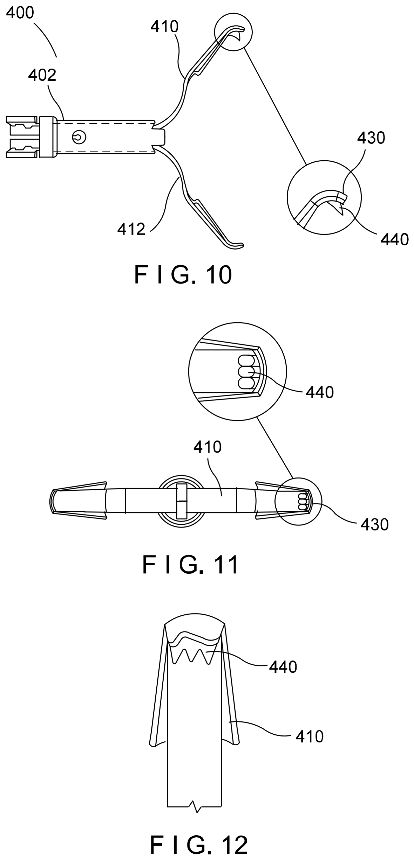

In addition to spikes, hooks and barbs, other geometries can be used to improve attachment of clip arms to the target tissue defect. For example, in another exemplary embodiment, a clip assembly 402 of a clip system 400, shown in Figs. 10-12, includes sharp teeth 440 at the distal tip 430 of at least one of first and second clip arms 410, 412. For example, as can be seen in Figs. 11-12, the first clip arm 410 includes three teeth 440 positioned at an inner surface of the distal tip 430 of the first clip arm 410. It is noted that while this embodiment includes three teeth, any number of teeth may be used, as would be understood by those skilled in the art. The teeth 440 have sharpened tips to allow for tissue piercing and retention. Additionally, the distal tip 430 of the first clip arm 410 may be angled at approximately 90 degrees relative to the remainder of the clip arm 410 to allow for tissue retention when the clip arms 410, 412 are reopened.

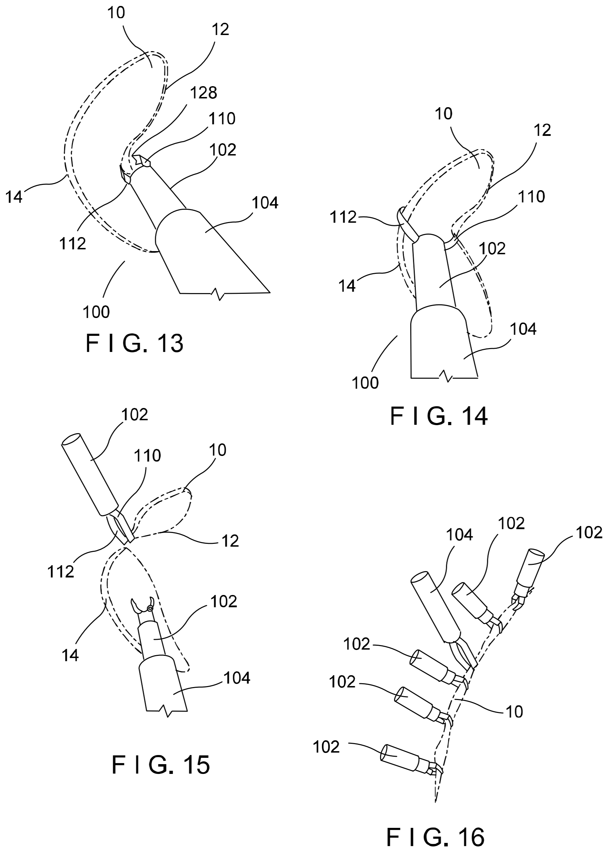

A method of use of system 100 according to an exemplary embodiment is depicted in Figs. 13-16. Initially, the clip assembly 102 is inserted through a working channel of an endoscope (or any other insertion device) and inserted into the body (e.g., through a natural body lumen) to a site adjacent to a target defect 10 of tissue to be clipped closed. The clip assembly 102 is inserted to the target tissue in the closed configuration to facilitate its passage through the working channel. Upon reaching the site of the target tissue defect, the clip assembly 102 is advanced out of the distal end of the working channel of the endoscope and the clip arms 110, 112 are extended out of the capsule 116 to move the clip arms 110, 112 to the open tissue receiving configuration.

Once a first edge 12 of the tissue defect target tissue has been received between the clip arms 110, 112, the clip arms 110, 112 are moved toward the closed configuration so that the spikes 128 pierce the first edge 12 of the tissue defect so that the first edge 12 is gripped between distal ends of the clip arms 110, 112. The clip arms 110, 112 are moved toward the tissue gripping configuration by drawing the control member proximally with respect to the capsule 116. Once the clip assembly 102 is in the closed tissue gripping configuration, the first edge 12 is dragged towards the second edge 14 of the defect via the clip assembly 102, via the applicator 104. When the first and second edges 12, 14 of the tissue defect are adjacent to one another, the clip arms 110, 112 are gently reopened. As the clip arms 110, 112 are reopened, the first arm 110 releases the first edge 12 of the tissue defect but the two spikes 128 of the second clip arm 112 keep the first edge 12 attached to the second clip arm 112. The clip arms 110, 112 are subsequently re-closed over both edges 12, 14 of the tissue defect and the first clip assembly 102 is deployed from the applicator 104 via the actuator. Additional clip assemblies 102 may be placed along the tissue edges to close the defect.

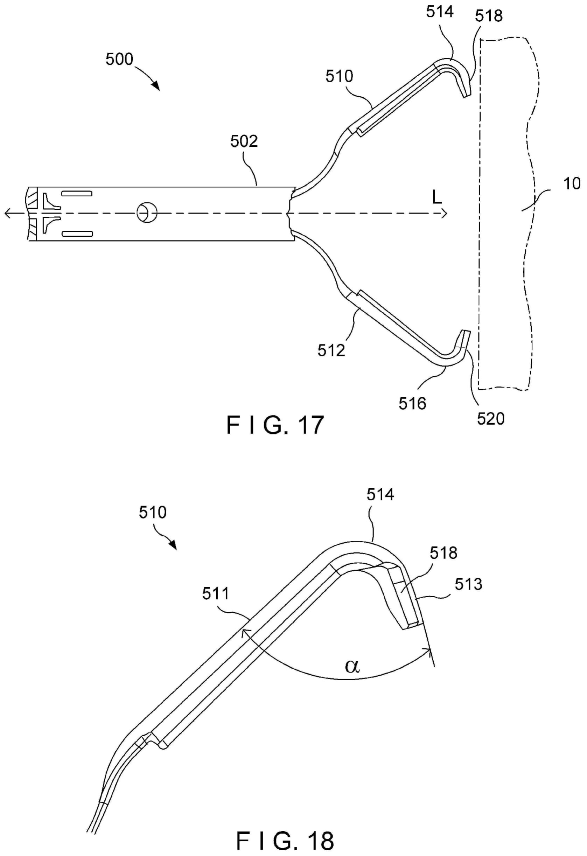

In another exemplary embodiment, shown in Figs. 17-24, a system 500 is similar to systems 100, 200, 300 and 400 except as described herein. The system 500 includes a clip assembly 502 with first and second clip arms 510, 512. As shown in Fig. 17, the clip assembly 502 is configured, so that, when addressing target tissue 10 in a substantially perpendicular approach (i.e., an approach in which a longitudinal axis L of the clipping assembly 502 is oriented substantially perpendicular to a surface of tissue 10 to be clipped) distal ends of the clip arms, when in an open tissue receiving configuration, extend at predetermined angles relative to the surface of the tissue 10 as will be described in more detail below.

As would be understood by those skilled in the art, although Fig. 17 shows the surface tissue 10 as substantially planar, this arrangement of a plane perpendicular to an angle of approach of the clip of the system 500 is employed to illustrate the configuration of the various components of the system 500 while the system 500 may be used to clip tissue of any of a variety of shapes of tissue surfaces from any desired angle of approach.

The first clip arm 510 has a distal curved portion 514 and a first tissue retention feature 518 extending laterally inward therefrom toward the axis L. Similarly, the second clip arm 512 has a distal curved portion 516 and a second tissue retention feature 520 extending laterally inward therefrom toward the axis L. In the exemplary embodiment, each of the first and second tissue retention features 518, 520, respectively, includes one or more teeth which are, in this embodiment, integrally formed by a bend in each of the first and second clip arms 510, 512, respectively, at the curved portions 514, 516, respectively, to extend toward the axis L. The retention features 518, 520 are angled inward toward the axis L at an angle selected so that, as the first and second clip arms 510, 512, respectively, are drawn toward one another into the tissue clipping configuration, tissue received between the clip arms 510, 512 is gripped by the first and second tissue retention features 518, 520.

As shown in Fig. 18, the curved portion 514 of the first clip arm 510 is defines an angle α between an exterior side 511 of the first clip arm 510 and an exterior side 513 of the retention feature 518. The angle α in this embodiment may range from 30° to 100°, however a preferred range is between 60° and 90°. The configuration of the second clip arm 512 is, in this embodiment, substantially a mirror image of the first clip arm 510. When the angle α is too acute, tissue may become stuck to the clip arm 510 before the user has decided to deploy and lock the clip over the target tissue. This may make it difficult to release the tissue and reposition the clip without potentially tearing tissue. Moreover, if the angle α is too obtuse, tissue grasping capabilities of the clipping assembly 502 may be compromised, potentially resulting in puncturing or tearing of tissue and/or prematurely releasing the target tissue grasped by the clipping assembly 502.

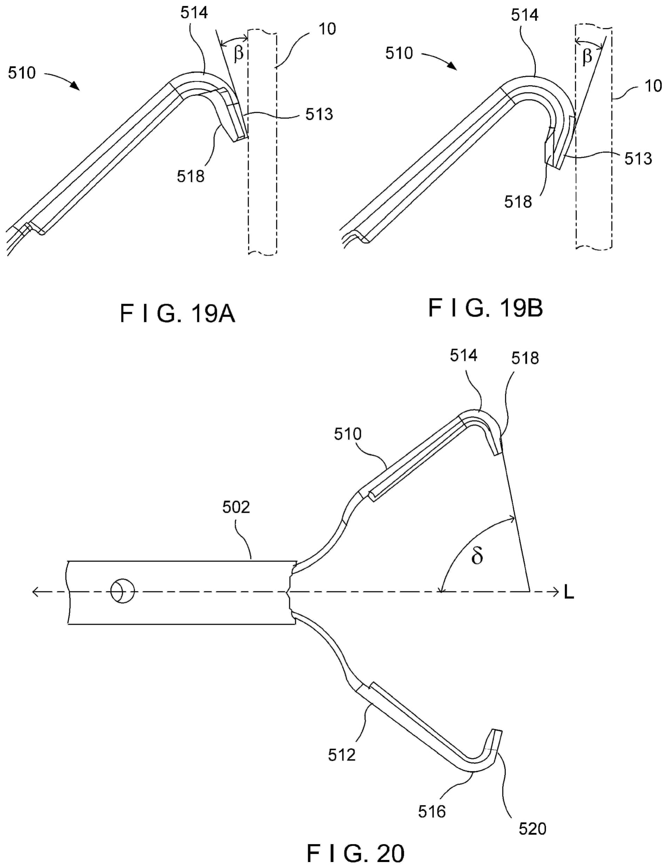

As mentioned above, the first and second clips arms 510, 512 spread apart from one another when freed to move into the open, tissue receiving configuration so that each forms an angle δ relative to the axis L which angle δ, in this embodiment, is the same for the first clip arm 510 as it is for the second clip arm 512. As shown in Figs. 19A and 19B, when the system 500 is positioned so that the axis L is perpendicular to the surface of the tissue 10 in the open, tissue receiving configuration, the first clip arm 510 forms an angle β relative to the surface of the tissue 10. The angle β is measured between the exterior side 513 of the retention feature 518 and a surface of the tissue 10. The angle β may have an exemplary range from -20° to 40°, however, in this embodiment, the range is preferably between 0° and 20°. In Fig. 19A, the first clip arm 510 forms a positive angle β (e.g., 20°) relative to the surface of the tissue 10, while in Fig. 19B, the clip arm 510 forms a negative angle β (e.g., -20°) relative to the surface of the tissue 10.

Additionally, as seen in Fig. 20, the clip arm 510 forms an angle δ with the longitudinal axis L of the clip assembly 502. The angle δ in this embodiment has a range from 50° to 110°, however, the angle δ in this embodiment may preferably be between 70° and 90°. As would be understood by those skilled in the art, the angle α, the angle β, and the angle δ are proportionally related to one another. Furthermore, in this embodiment, the first and second clip arms 510, 512 are formed as substantial mirror images of one another with respect to the angles α, β and δ. However, those skilled in the art will understand that any of these angles may be varied from one clip arm to another within the exemplary ranges as desired.

In the exemplary embodiment, the retention features 518, 520 are designed to conform to one another, such that, when the clipping assembly 502 is inserted through a working channel of an endoscope in the closed configuration, sharp edges of the retention features 518, 520 are shielded to avoid any edge of any of the retention features protruding beyond an outer limit of the clip arms 510, 512 (i.e., no edge of any of the retention features 518, 520 protrudes further toward a wall of a cylindrical lumen within which the clip assembly 502 is received than do the smooth radially outer surfaces of the clip arms 510, 512). This shielding mitigates the risk that any sharp edge of the clip arms 510, 512 will engage and damage any portion of a working channel of an endoscope through which the clipping assembly 502 is deployed. Additionally, the shielding mitigates the risk that, for example, a pouch within which the product is stored, will be torn by any of these sharp surfaces potentially compromising product sterility.

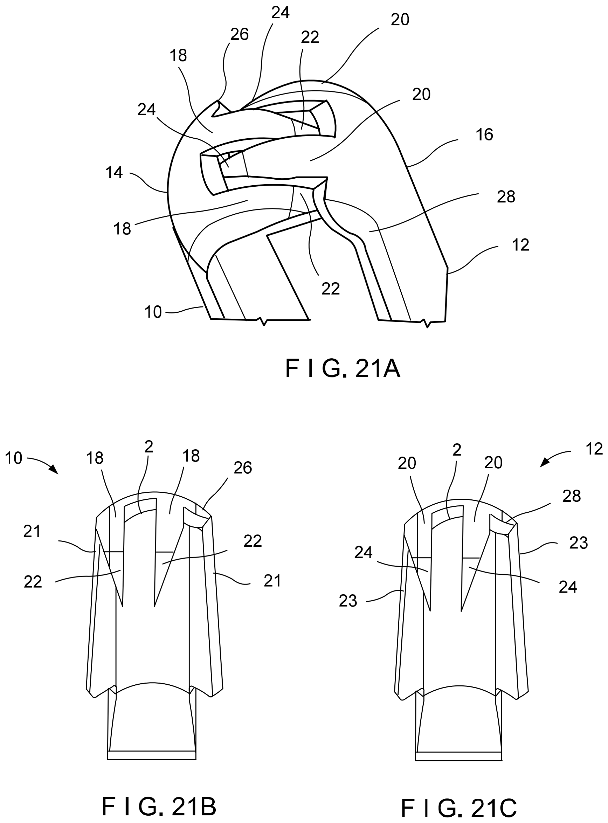

As shown in Figs. 21A-21C, the retention features 518, 520 in this embodiment correspond to one another so that, when they are drawn together into the tissue clipping configuration, each of the teeth of the first clip arm 510 faces a correspondingly sized gap between the teeth of the second clip arm 512 so that they nest together as described below. In this embodiment, each of the retention features 518 has a sharpened tip 522 while each of the retention features 520 has a sharpened tip 524 to facilitate the piercing and retention of engaged tissue. In the exemplary embodiment, the retention feature 518 includes a first tooth 518a having a first sharpened tip 522a and a second tooth 518b having a second sharpened tip 522b, as shown in Fig. 21B.

For the retention feature 518, the first tooth 518a is positioned adjacent to a first lateral edge 521a of the clip arm 510 while the second tooth 518b is positioned more centrally and separated from the first tooth 518a by a first gap 525 having a width substantially corresponding to a width (a direction across the sharpened tip from the first lateral edge to the second lateral edge of the corresponding clip arm) of a second sharpened tip 524b of the clip arm 512. The second tooth 518b is spaced from a second lateral edge 521b by a distance substantially corresponding to a width of a first sharpened tip 524a of the second clip arm 512. The retention feature 518 further includes a first rounded overhang 526 adjacent to the second tooth 518b, the first overhang 526 acting as a guard to shield the first sharpened tip 524a. The first overhang 526 extends distally from the curved portion 514 a partial length of the second tooth 518b.

Similarly to the retention features 518, the retention feature 520 includes a first tooth 520a having the first sharpened tip 524a and a second tooth 520b having the second sharpened tip 522b, as shown in Fig. 21C. For the retention feature 520, the first tooth 520a is positioned adjacent to a first lateral edge 523a of the clip arm 512 while the second tooth 520b is positioned more centrally separated from the first tooth 520a via a gap 527 having a width substantially corresponding to a width of the second sharpened tip 522b of the first clip arm 510. The second tooth 520b is spaced from the second lateral edge 523b by a distance substantially corresponding to a width of the first sharpened tip 522a of the first clip arm 510. The retention feature 520 further includes a second rounded overhang 528 adjacent to the second tooth 520b, the second overhang 528 acting as a guard to shield the first sharpened tip 522a. The second overhang 528 extends distally from the curved portion 516 a partial length of the second tooth 520b.

As one skilled in the art will understand, a width of the first clip arm 510 (i.e., a distance from the first lateral edge 521a to the second lateral edge 521b) is substantially similar to a width of the second clip arm 512 (i.e., a distance from the first lateral edge 523a to the second lateral edge 523b) so that the teeth of one of the clip arms are always shielded by the opposite clip arm. That is, where a number of teeth in one of the clip arms is the same as on the opposite arm, or, as here, where the teeth are positioned off the center of the clip arm, the distal portion of each clip arm will have a width selected to extend, in the closed configuration at least as far laterally as required to shield all of the teeth of the opposite clip arm.

This configuration allows the second sharpened tip 522b, in the closed configuration, to be received in the gap 527 between the first and second teeth 520a, 520b while the first sharpened tip 522a is received under the second overhang 528. Similarly, in the closed configuration, the second sharpened 524b is received in the gap 525 between the first and second teeth 518a, 518b while the first sharpened tip 524a is received under the first overhang 526 of the first clip arm 510, as shown in Fig. 21A. This configuration provides shielding, such that the sharpened tips 522, 524 of the retention features 518, 520 do not extend past the lateral edges 523a, 523b of the second clip arm 512 and the lateral edges 521a, 521b of the first clip arm 510.

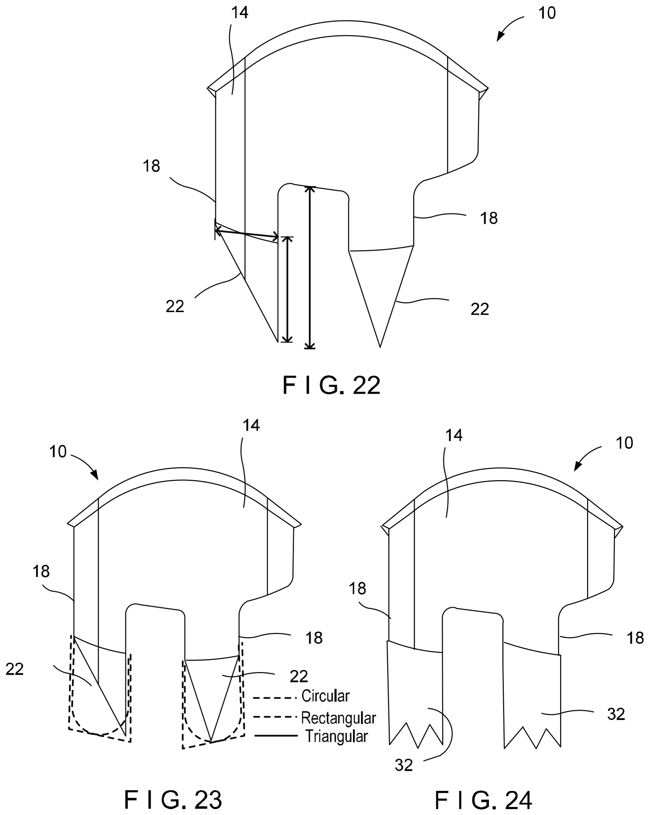

Sharpness of the retention features 518, 520 is determined by the sharpened tips 522, 524. In the exemplary embodiment, the sharpened tips 522, 524 are generally triangular. As shown in Fig. 22, the second tooth 518b and the second sharpened tip 522b are substantially similar to the first tooth 518a and the first sharpened tip 522a. A sharpness of the first tooth 518a (as well as teeth 518b, 520a, and 520b) may be expressed as a first ratio between a width W of the tooth 518a and a length T of the tooth 518a. As will be understood by those skilled in the art, a length V of the first sharpened tip 522a is part of the length T of the tooth 518a. The first ratio (T/W) is expressed as the length T over the width W.

The first ratio in this embodiment has an exemplary range from 0.5 to 6, however a preferred range is between 2 and 4. Further, a sharpness of the tooth 518a may be expressed as a second ratio between the width W and the length V is part of the length T of the tooth 518a. The second ratio (V/W) is expressed as the length V over the width W. The second ratio has, in this embodiment, an exemplary range from 0.5 to 6 and a preferred range of between 2 and 3. In the exemplary embodiment, the sharpened tips 522, 524 are triangular in shape, however the sharpened tips 522, 524 may be a variety of other shapes (e.g., circular, rectangular, etc.), as in Fig. 23. Additionally, instead of the sharpened tips 522, 524, the retention features 518, 520 may be formed as a plurality of barbs 532a, 532b, as shown in Fig. 24. It is noted that while these embodiments include two teeth on each clip arm, the number of teeth per clip arm may be varied as desired.

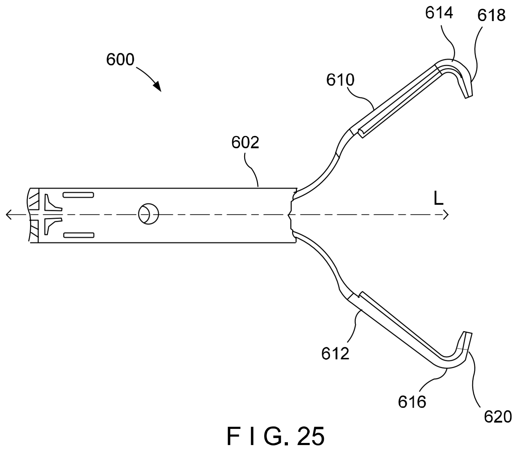

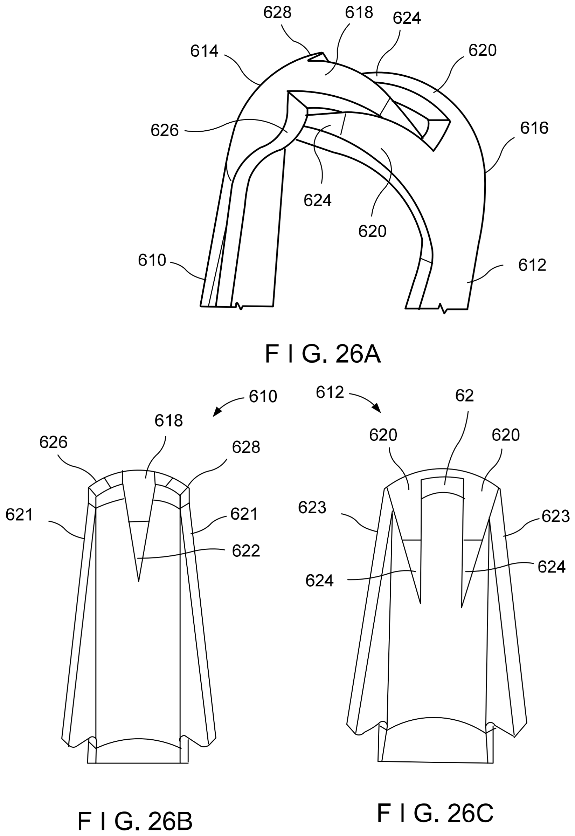

In another exemplary embodiment, shown in Figs. 25-26C, a system 600 is substantially the same as systems 100, 200, 300, 400 and 500 except as described herein. Similar to the system 500, the system 600 includes a clip assembly 602 with first and second clip arms 610, 612. As shown in Fig. 25, the first clip arm 610 has a curved portion 614 and a first tissue retention feature 618 extending distally therefrom, the retention feature 618 having. Similarly, the second clip arm 612 has a curved portion 616 and a second tissue retention feature 620 extending distally therefrom. The retention features 618, 620 are configured to grasp target tissue therebetween when the clip assembly 602 is moved into a closed configuration.

As shown in Figs. 26A-26C, similar to the retention features 518, 520 of the clipping assembly 502, the retention features 618, 620 of the clipping assembly 602 are designed to conform to one another. When the clipping assembly 602 is inserted through a working channel of an endoscope in the closed configuration, the retention features 618, 620 are shielded to avoid any edge of the retention features 618, 620 protruding beyond an outer limit of the clip arms 610, 612 (i.e., no edge of any of the retention features 618, 620 protrudes further toward a wall of a cylindrical lumen within which the clip assembly 602 is received than do the smooth radially outer surfaces of the clip arms 610, 612) to mitigates the risk that any sharp edge will engage and damage any portion of a working channel of an endoscope through which the clipping assembly 602 is deployed.

The clipping assembly 602 differs from the clipping assembly 502 in that the retention feature 618 is different from the retention feature 620, while the retention feature 518 is substantially similar to the retention feature 520. Specifically, each of the retention features 518, 520 has the same number of teeth (e.g., the retention feature 518 has two teeth and the retention feature 520 has two teeth), while the number of teeth of the retention features 618 differs from the number of teeth of the retention feature 620 (e.g., the retention feature 618 has one tooth and the retention feature 620 has two teeth), as described below. Accordingly, the retention feature 618 has a single sharpened tip 622 while the retention feature 620 has two sharpened tips 624 to facilitate the piercing and retention of tissue engaged by the retention features 618, 620. As would be understood by those skilled in the art, any of the various configurations of the retention features described herein may be employed with clip arms having any of the angles α, β and δ described above.

In the exemplary embodiment, sharpness of the retention features 618, 620 is determined by the sharpened tips 622, 624, the sharpness of the retention features 618, 620 being determined similarly to the sharpness of the retention features 518, 520. Additionally, in the exemplary embodiment, the sharpened tips 622, 624 are triangular in shape, however the sharpened tips 622, 624 may be a variety of other shapes (e.g., circular, rectangular, etc.), similar to the sharpened tips 522, 524 in Figs. 22-24. When the clipping assembly 602 is in the closed configuration, the sharpened tip 622, 624 are shielded as described below.

In the exemplary embodiment, the retention feature 618 includes a single tooth 618a having a single sharpened tip 622a, as shown in Fig. 26B. For the retention feature 618 in this embodiment, the tooth 618a is positioned centrally with respect to the lateral sides of the first clip arm 610 on the distal end of the curved portion 614. The retention feature 618 further includes a first rounded overhang 626 acting as a guard to shield a second sharpened tip 624b of the clip arm 612, and a second rounded overhang 628 acting as a guard to shield a first sharpened tip 624a of the second clip arm 612.

The first overhang 626 is between a first lateral edge 621a of the first clip arm 610 and the tooth 618a, while the second overhang 628 is between a second later edge 621b of the clip arm 610 and the tooth 618a. The first and second overhangs 626, 628 extend distally from the curved portion 614 a partial length of the tooth 618. The first overhang 626 has a width substantially corresponding to a width (a direction across the sharpened tip from one of the first lateral edge to the second lateral edge of the corresponding clip arm) of the second sharpened tip 624b. Similarly, the second overhang 628 has a width substantially corresponding to a width of the first sharpened tip 624a.

In the exemplary embodiment, the retention feature 620 includes a first tooth 620a having the first sharpened tip 624a and a second tooth 620b having the second sharpened tip 624b, as shown in Fig. 26C. For the retention feature 620, the first tooth 620a is positioned adjacent to a first lateral edge 623a of the second clip arm 612 while the second tooth 620b is positioned adjacent to a second lateral edge 623b. The first tooth 620a and the second tooth 620b are separated from one another via a gap 625 having a width substantially corresponding to a width of the sharpened tip 622a of the first clip arm 610.

As one skilled in the art will understand, a width of the first clip arm 610 (i.e., a distance from the first lateral edge 621a to the second lateral edge 621b) is substantially similar to a width of the second clip arm 612 (i.e., a distance from the first lateral edge 623a to the second lateral edge 623b) so that the teeth of one of the clip arms are always shielded by the opposite clip arm. That is, where a number of teeth in one of the clip arms is less than on the opposite arm, or, as here, where the teeth are positioned off the center of the clip arm, the distal portion of each clip arm will have a width selected to extend, in the closed configuration at least as far laterally as required to shield all of the teeth of the opposite clip arm. This configuration allows the sharpened tip 624a, in the closed configuration, to be received under the second overhang 628 to prevent the sharpened tip 624a from extending past the first clip arm 610.

Similarly, the sharpened tip 624b is received under the first overhang 626 to prevent the sharpened tip 624b from extending past the first clip arm 610. Moreover, the sharpened 622a is received in the gap 625 between the first and second teeth 620a and 620b to prevent the sharpened tip 622a from extending past the second clip arm 612, as shown in Fig. 26A. This configuration provides shielding, such that the sharpened tips 622, 624 of the retention features 618, 620 do not extend past the lateral edges 623a, 623b of the second clip arm 612 and the lateral edges 621a, 621b of the first clip arm 610.

As would be understood by those skilled in the art, although these embodiments include a clip arm with one tooth and a clip arm with two teeth, each of the clip arms may have any number of teeth as long as the number of teeth on each of the clip arms is different. A method of use of systems 500 and 600 is substantially the same as that described above for system 100. It will be appreciated by those skilled in the art that changes may be made to the embodiments described above without departing from the inventive concept thereof. It should further be appreciated that structural features and methods associated with one of the embodiments can be incorporated into other embodiments.

It is understood, therefore, that this disclosure is not limited to the particular embodiment disclosed, but rather modifications are also covered within the scope of the present invention as defined by the appended claims.

The following aspects are preferred embodiments of the invention:
1. A device for treating tissue, comprising:
   a capsule extending longitudinally from a proximal end to a distal end and including a channel extending therethrough along a longitudinal axis; and
   first and second clip arms, each extending from a proximal end to a distal end, the proximal ends being received within the channel of the capsule to be moved between an open tissue receiving configuration, in which the distal ends of the clip arms are separated from one another, and a closed tissue clipping configuration, in which the distal ends of the clip arms are moved toward one another,
   wherein the first clip arm includes a first portion which, in the tissue clipping configuration extends substantially parallel to a first portion of the second arm and parallel to the longitudinal axis, the first clip arm including a first tissue retention feature extending radially inward from the first portion of the first clip arm toward the longitudinal axis, the first tissue retention feature being configured to grip target tissue when in the tissue clipping configuration and subsequently release the target tissue when the first and second clip arms are moved toward the open tissue receiving configuration, the first retention feature being coupled to the first portion of the first clip arm via a first curved portion and wherein the second clip arm includes a second tissue retention feature extending radially inward from the first portion of the second clip arm toward the longitudinal axis, the second tissue retention feature being configured to grip target tissue when in the tissue clipping configuration and subsequently release the target tissue when the first and second clip arms are moved toward the open tissue receiving configuration, the second retention feature being coupled to the first portion of the second clip arm via a second curved portion.
2. The device of aspect 1, wherein the first tissue retention feature includes a first tooth extending radially inward from the first curved portion and the second tissue retention feature includes a second tooth extending radially inward toward the longitudinal axis from the second curved portion.
3. The device of aspect 2, wherein the first retention feature has a number of teeth that differs from a number of teeth of the second retention feature.
4. The device of aspect 2, wherein the first retention feature includes a first guard positioned and shaped to overhang a sharpened tip of the second tooth so that the sharpened tip of the second tooth is, when the first and second clip arms are in the tissue clipping configuration, proximal of the first guard and closer to the longitudinal axis than lateral edges of the first and second clip arms.
5. The device of aspect 2, wherein the second retention feature includes a second guard positioned and shaped to overhang a sharpened tip of the first tooth so that the sharpened tip of the first tooth is, when the first and second clip arms are in the tissue clipping configuration, proximal of the second guard and closer to the longitudinal axis than lateral edges of the first and second clip arms.
6. The device of any one of aspects 1-5, wherein the first retention feature extends at a first angle relative to the first portion of the first clip arm of 30 degrees to 100 degrees and the second retention feature extends at a second angle relative to the first portion of the second clip arm of 30 degrees to 100 degrees.
7. The device of aspect 6, wherein the first angle is equal to the second angle.
8. The device of any one of aspects 1-7, wherein the first retention feature includes first sharpened tips extending radially inward therefrom and the second retention feature includes second sharpened tips extending radially inward therefrom.
9. The device of aspect 8, wherein the first sharpened tips and the second sharpened tips are one of triangular, circular, or rectangular in shape.
10. The device of any one of aspects 1-9, wherein the first retention feature includes a first plurality of barbs extending radially inward therefrom and the second retention feature includes a second plurality of barbs extending radially inward therefrom.
11. A reloadable clip system, comprising:
   an applicator including a catheter and a control member extending therethrough, the control member extending from a proximal end to a distal end and being longitudinally movable relative to the applicator;
   at least one clip assembly coupled to the applicator, each clip assembly comprising:
      a capsule extending longitudinally from a proximal end to a distal end and including a channel extending therethrough along a longitudinal axis; and
      first and second clip arms each extending from a proximal end to a distal end, the proximal ends being received within the channel of the capsule to be moved between an open tissue receiving configuration, in which the distal ends of the clip arms are separated from one another, and a closed tissue clipping configuration, in which the distal ends of the clip arms are moved toward one another,
      wherein the first clip arm includes a first portion which, in the tissue clipping configuration extends substantially parallel to a first portion of the second arm and parallel to the longitudinal axis, the first clip arm including a first tissue retention feature extending radially inward from the first portion of the first clip arm toward the longitudinal axis, the first tissue retention feature being configured to grip target tissue when in the tissue clipping configuration and subsequently release the target tissue when the first and second clip arms are moved toward the open tissue receiving configuration, the first retention feature being coupled to the first portion of the first clip arm via a first curved portion and wherein the second clip arm includes a second tissue retention feature extending radially inward from the first portion of the second clip arm toward the longitudinal axis, the second tissue retention feature being configured to grip target tissue when in the tissue clipping configuration and subsequently release the target tissue when the first and second clip arms are moved toward the open tissue receiving configuration, the second retention feature being coupled to the first portion of the second clip arm via a second curved portion.
12. The system of aspect 11, wherein the first tissue retention feature includes a first tooth extending radially inward from the first curved portion and the second tissue retention feature includes a second tooth extending radially inward toward the longitudinal axis from the second curved portion.
13. The system of aspect 12, wherein the first retention feature includes a first guard positioned and shaped to overhang a sharpened tip of the second tooth so that the sharpened tip of the second tooth is, when the first and second clip arms are in the tissue clipping configuration, proximal of the first guard and closer to the longitudinal axis than lateral edges of the first and second clip arms.
14. The system of any one of aspects 11-13, wherein the first retention feature extends at a first angle relative to the first portion of the first clip arm of 30 degrees to 100 degrees and the second retention feature extends at a second angle relative to the first portion of the second clip arm of 30 degrees to 100 degrees.
15. The system of any one of aspects 11-14, wherein the second retention feature includes a second guard positioned and shaped to overhang a sharpened tip of the first tooth so that the sharpened tip of the first tooth is, when the first and second clip arms are in the tissue clipping configuration, proximal of the second guard and closer to the longitudinal axis than lateral edges of the first and second clip arms.

## Claims

1. A device for treating tissue, comprising:
a capsule extending longitudinally from a proximal end to a distal end and including a channel extending therethrough along a longitudinal axis; and
first and second clip arms, each extending from a proximal end to a distal end, the proximal ends being received within the channel of the capsule to be moved between an open tissue receiving configuration, in which the distal ends of the clip arms are separated from one another, and a closed tissue clipping configuration, in which the distal ends of the clip arms are moved toward one another,
wherein the first clip arm includes a first portion which, in the tissue clipping configuration extends substantially parallel to a first portion of the second clip arm and parallel to the longitudinal axis, the first clip arm including a first tissue retention feature, the first tissue retention feature including a first clip arm first tooth and a first clip arm second tooth, each extending radially inward from the first portion of the first clip arm toward the longitudinal axis,
wherein the second clip arm includes a second tissue retention feature, the second tissue retention feature including a second clip arm second tooth extending radially inward from the first portion of the second clip arm toward the longitudinal axis; and
wherein the first tooth of the first clip arm is positioned adjacent to a first lateral edge of the first clip arm while the second tooth of the first clip arm is positioned more centrally and separated from the first tooth of the first clip arm by a first gap having a width substantially corresponding to a width of a second sharpened tip of the second tooth of the second clip arm.

2. The device of claim 1, wherein the first clip arm first tooth is coupled to the first portion of the first clip arm via a first curved portion and the second clip arm second tooth is coupled to the first portion of the second clip arm via a second curved portion.

3. The device of claim 1, wherein the first retention feature has a number of teeth that differs from a number of teeth of the second retention feature.

4. The device of claim 1, wherein the first retention feature includes a first guard positioned and shaped to overhang a sharpened tip of the second clip arm second tooth so that the sharpened tip of the second clip arm second tooth is, when the first and second clip arms are in the tissue clipping configuration, proximal of the first guard and closer to the longitudinal axis than lateral edges of the first and second clip arms.

5. The device of claim 1, wherein the second retention feature includes a second guard positioned and shaped to overhang a sharpened tip of the first clip arm first tooth so that the sharpened tip of the first clip arm first tooth is, when the first and second clip arms are in the tissue clipping configuration, proximal of the second guard and closer to the longitudinal axis than lateral edges of the first and second clip arms.

6. The device of any one of claims 1-5, wherein the first retention feature extends at a first angle relative to the first portion of the first clip arm of 30 degrees to 100 degrees and the second retention feature extends at a second angle relative to the first portion of the second clip arm of 30 degrees to 100 degrees.

7. The device of claim 6, wherein the first angle is equal to the second angle.

8. The device of any one of claims 1-7, wherein the first clip arm first tooth includes a first sharpened tip and the second clip arm second tooth includes a second sharpened tip.

9. The device of claim 8, wherein the first sharpened tip and the second sharpened tip are one of triangular, circular, or rectangular in shape.

10. The device of any one of claims 1-9, wherein the first retention feature includes a first plurality of barbs extending radially inward therefrom and the second retention feature includes a second plurality of barbs extending radially inward therefrom.

11. The device of claim 1, wherein a width of the first clip arm is substantially similar to a width of the second clip arm so that the tooth of either clip arm is shielded by the opposite clip arm.

12. The device of claim 1, wherein the first and second teeth are positioned off of a longitudinal center of the first and second clip arms, respectively, and the distal portion of each clip arm will have a width selected to extend laterally in the closed tissue clipping configuration so as to shield the tooth of the opposite clip arm.

13. A reloadable clip system, comprising:
an applicator including a catheter and a control member extending therethrough, the control member extending from a proximal end to a distal end and being longitudinally movable relative to the applicator;
at least one clip assembly coupled to the applicator, each clip assembly comprising:
a capsule extending longitudinally from a proximal end to a distal end along a longitudinal axis; and
first and second clip arms each extending from a proximal end to a distal end, the proximal ends being received within the capsule to be moved between an open tissue receiving configuration, in which the distal ends of the clip arms are separated from one another, and a closed tissue clipping configuration, in which the distal ends of the clip arms are moved toward one another,
wherein the first clip arm includes a first portion which, in the tissue clipping configuration extends substantially parallel to a first portion of the second arm and parallel to the longitudinal axis, the first clip arm including a first tissue retention feature, the first tissue retention feature including a first tooth extending radially inward from the first portion of the first clip arm toward the longitudinal axis, and wherein the second clip arm includes a second tissue retention feature, the second tissue retention feature including a second tooth extending radially inward from the first portion of the second clip arm toward the longitudinal axis; and
wherein the first tooth of the first clip arm is positioned adjacent to a first lateral edge of the first clip arm while the second tooth of the first clip arm is positioned more centrally and separated from the first tooth of the first clip arm by a first gap having a width substantially corresponding to a width of a second sharpened tip of the second tooth of the second clip arm.

14. The system of claim 13, wherein the first retention feature extends at a first angle relative to the first portion of the first clip arm of 30 degrees to 100 degrees and the second retention feature extends at a second angle relative to the first portion of the second clip arm of 30 degrees to 100 degrees.

15. The system of claim 13 or claim 14, wherein the second retention feature includes a second guard positioned and shaped to overhang a sharpened tip of the first tooth so that the sharpened tip of the first tooth is, when the first and second clip arms are in the tissue clipping configuration, proximal of the second guard and closer to the longitudinal axis than lateral edges of the first and second clip arms.
